# EUROPÄISCHE PATENTSCHRIFT

(11) **EP 0 502 813 B1**
(45) Veröffentlichungstag und Bekanntmachung des Hinweises auf die Patenterteilung: **13.07.1994**
(21) Anmeldenummer: 92810075.9
(22) Anmeldetag: 04.02.1992
(51) Int. Cl.: B65D 81/06, A61F 2/00

(54) **Reinstraumverpackung**
Sterile packaging
Emballage sterile

(30) Priorität: 01.03.1991 CH 631/91
(43) Veröffentlichungstag der Anmeldung: 09.09.1992
(73) Patentinhaber: SULZER Medizinaltechnik AG, 8404 Winterthur (CH)
(72) Erfinder: Artusi, Aldo, CH-8466 Trüllikon (CH); Artusi, Reto, CH-8466 Trüllikon (CH)
(74) Vertreter: Hammer, Bruno, Dr.

(56) Entgegenhaltungen:
- EP-A- 0 111 062
- WO-A-86/06044
- GB-A- 838 143
- US-A- 3 918 581

## Beschreibung

Die Erfindung handelt von einer Reinstraumverpackung für einen länglichen starren Körper, die aus einer Trägerschale aus Kunststoff, welche luftdicht mit einer Deckelfolie verschliessbar ist, und die aus Stützkörpern aus kompressiblem Schaumstoff besteht.

Verpackungen, die wie Joghurtbecher aus einer Trägerschale aus Kunststoff und aus einer luftdicht verschliessbaren Deckelfolie bestehen sind aus der Nahrungsmittelindustrie bekannt. Auch in der Reinstraumtechnik werden luftdichte Verpackungen verwendet, die beim Abpacken von starren und empfindlichen Körpern zusätzliche Stützkörper aus kompressiblem Schaumstoff aufweisen, die das abgepackte Produkt vor Stössen schützen sollen. So zeigt die Patentschrift EP-A- 0 111 062 eine sterilisierbare Doppelverpackung für Endoprothesen, deren innerer Behälter eine Reinstraumverpackung gemäß dem Oberbegriff des Anspruchs 1 darstellt.

Die Anwendung dieser Verpackungstechnik für Implantate, die bei Gelenkprothesen aus länglichen starren Körpern bestehen, birgt insofern Probleme in sich als das Implantat in jede Richtung abgestützt sein sollte, als es im verpackten Zustand identifizierbar sein sollte und als es beim Herausnehmen unmittelbar neben dem Operationsfeld keinerlei Fremdkörper wie Teile von Stützkörpern mitnehmen sollte.

Diesen Umständen trägt die Erfindung Rechnung. Sie löst die Aufgabe, eine allseitig stoss-sichere Reinstraumverpackung für unterschiedlich grosse längliche und starre Körper zu schaffen, die ein keimfreies Verpacken erlaubt und die beim Herausnehmen des starren Körpers eine zusammenhängende Leerpackung hinterlässt. Gemäss der Erfindung wird die Aufgabe dadurch gelöst, dass kopf- und fuss-seitige Stützkörper als Trennwand den Kontakt des starren Körpers in Richtung der Längsachse zur Trägerschale verhindern, dass mittige Stützkörper für die Lagerung des starren Körpers in Richtung der Längsachse der Trägerschale jeweils einen Durchbruch aufweisen, der über einen Schlitz auf der Deckelseite zugänglich ist, und dass die Stützkörper quer zur Längsachse der Trägerschale und in Richtung Deckel mit Vorsprüngen in seitlichen Taschen der Trägerschale verankert sind.

Die Vorteile der Erfindung sind darin zu sehen, dass im sterilen Bereich eine einzige Person ohne das Risiko von umherfliegendem Verpackungsmaterial die Verpackung öffnen und den starren Körper greifen und in ein Arbeitsfeld bringen kann. Die abhängigen Ansprüche 2 bis 8 beziehen sich auf vorteilhafte Weiterbildungen der Erfindung.

Im folgenden wird die Erfindung anhand von einem Ausführungsbeispiel beschrieben. Es zeigen:
- Fig. 1: schematisch die perspektivische Ansicht einer Trägerschale in die die Stützkörper zur Aufnahme eines länglichen starren Körpers eingesetzt sind; und
- Fig. 2: schematisch einen Querschnitt auf Höhe eines mittigen Stützkörpers durch eine verschlossene Verpackung.

In den Figuren ist eine Reinstraumverpackung für einen länglichen starren Körper gezeigt, die aus einer Trägerschale aus Kunststoff, welche luftdicht mit einer Deckelfolie verschliessbar ist, und die aus Stützkörpern aus kompressiblem Schaumstoff besteht. Die Stützkörper sind quer zur Längsachse der Trägerschale und in Richtung Deckel mit Vorsprüngen in seitlichen Taschen der Trägerschale verankert. Die kopf- und fuss-seitigen Stützkörper verhindern als Trennwand den Kontakt des starren Körpers in Richtung der Längsachse zur Trägerschale. Mittige Stützkörper für die Lagerung des starren Körpers weisen in Richtung der Längsachse der Trägerschale jeweils einen Durchbruch auf, der über einen Schlitz auf der Deckelseite zugänglich ist.

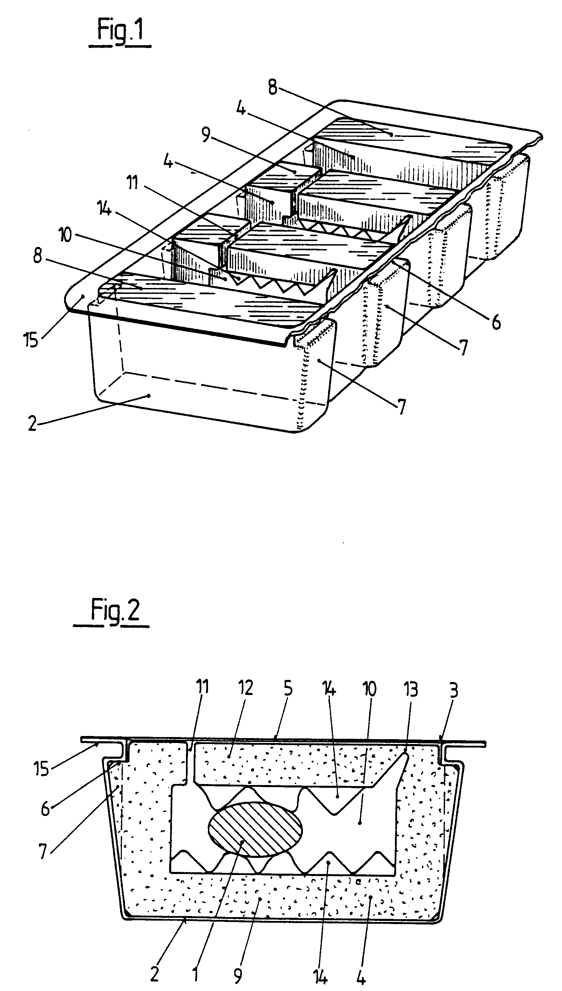

In Figur 1 ist eine Trägerschale 2 mit rechteckiger Grundfläche gezeigt, die am oberen Rand in einen in einer Ebene liegenden zusammenhängenden Kragen 15 ausmündet. Der Kragen 15 ist soweit seitlich über die darunterliegende Trägerschale 2 herausgezogen, dass ein maschinelles Versiegeln durch Verschweissen oder Verkleben mit einer Deckelfolie 3 möglich ist. Quer zur Längsachse der rechteckigen Grundfläche sind Stützkörper 4 in der Trägerschale 2 eingelegt, die mit Vorsprüngen 6 in seitlichen Taschen 7 der Seitenwände der Trägerschale 2 einrasten. Die kopf-und fuss-seitigen Stützkörper 4 bilden jeweils eine Trennwand 8, um den direkten Kontakt zwischen dem starren Körper 1 in Richtung der Längsachse zur Trägerschale 2 zu verhindern, während mittige Stützkörper 9 für die Lagerung des starren Körpers 1 in Richtung der Längsachse der Trägerschale 2 jeweils einen Durchbruch 10 aufweisen, der über einen Schlitz 11 auf der Deckelseite zugänglich ist. Wie in Figur 2 ersichtlich bilden die mittigen Stützkörper 9 zum Deckel 5 hin ausklappbare Haltelappen 12, die sich zwischen Schlitz 11 und scharnierähnlichen Gelenken 13, welche durch eine Querschnittreduktion in den oberen Ecken gebildet werden, erstrecken. Zum Einlegen der starren Körper wie z.B. von den Schäften von Hüftgelenksprothesen werden die Haltelappen 12 aus der Trägerschale 2 herausgeklappt. Der Widerstand der Haltelappen 12 gegen das Herausklappen in Richtung des Deckels 5 ist kleiner als der Widerstand der Vorsprünge 6 gegen das Herauslösen der Stützkörper 4 aus der Trägerschale 2. Nach dem Einlegen des starren Körpers 1 werden die Haltelappen 12 zurückgeklappt und zusammen mit dem Kragen 15 durch Deckelfolie 3 überdeckt. Zum Verbinden der Deckelfolie 3 mit dem Kragen 15 werden zunächst die Deckelfolie 3 und die darunterliegenden Haltelappen 12 soweit mit einem Stempel (nicht gezeigt) zusammengedrückt bis die Deckelfolie 3 in der Ebene des Kragens 15 liegt. Der Stempel hält die Deckelfolie 3 solange in dieser Position bis die Verbindung mit dem Kragen 15 erfolgt ist. Als Verbindung eignen sich Folienschweissen oder Kleben.

Der starre Körper 1 weist zu den Durchbrüchen 10 ein Uebermass auf, das durch eine Deformation der mittigen Stützkörper 9 an den Berührungspunkten elastisch kompensiert wird. Gleichzeitig entstehen durch die Klemmung Reibungskräfte, die ein Gleiten des starren Körpers 1 verhindern. Der starre Körper 1 ist durch die Klemmung elastisch federnd aufgehängt. Stösse quer zur Längsachse werden durch die Deformation der mittigen Stützkörper 9 abgefangen. Um nun die Klemmung für Stösse in der Längsachse nicht übermässig zu beanspruchen sind die kopf- und fuss-seitig als Trennwände 8 ausgeführten Stützkörper in unterschiedlicher Wandstärke in die Trägerschale 2 einsetzbar, damit der starre Körper 1 beim Einlegen formschlüssig in der Längsrichtung der Trägerschale 2 fixiert ist. Der starre Körper ist somit allseitig federnd und stoss-sicher aufgehängt und es besteht keine Gefahr, dass er bei einem Stoss Leerräume durchfliegt und die Trägerschale 2 durchstösst. Um mittige Stützkörper 9 für starre Körper 1 mit unterschiedlicher Dicke einsetzen zu können, weisen die Durchbrüche 10 nach innen gerichtete Vorsprünge 14 auf, den den Klemmbereich auch auf weniger dicke Teile ausdehnen. Die Vorsprünge 14 können als in Richtung der Längsachse verlaufende Rippen oder als von der Oberfläche nach innen wegstehende Nocken ausgeführt sein. Wichtig ist, dass der Schaumstoff der Stützkörper keinerlei lose oder schwach befestigte Partikel aufweist, die beim Auspacken mitgerissen werden könnten. Geschlossen-porige Schaumstoffe haben sich in dieser Richtung bewährt. Weiterhin ist es vorteilhaft die Lage der Beschriftung auf dem starren Körper 1 und die Abstände zwischen den Stützkörpern 4 so einander anzupassen, dass mit der Verwendung von transparenten Kunststoffen für Trägerschale und Deckelfolie 3 eine Identifikation im verpackten Zustand möglich ist. Für die Handhabung und Identifikation im nicht-sterilen Bereich ist die versiegelte Reinstraumverpackung zusätzlich steril in einer zweiten Verpackung z.B. in einem transparenten Aufreissbeutel verpackt. Die Uebergabe in den sterilen Arbeitsbereich erfolgt nach dem Aufreissen des Beutels an eine im sterilen Bereich arbeitende Person, die die Deckelfolie 3 soweit von der Trägerschale 2 abzieht bis die Haltelappen 12 und der starre Körper 1 freigegeben sind.

Zur besseren Verteilung der Kräfte im versiegelten Zustand, die durch die Klemmung von den mittigen Stützkörpern 9 auf die Deckelfolie 3 übertragen werden, kann die Deckelfolie auf der Innenseite im Abdeckbereich eine mit ihr verbundene starrere Druckplatte aufweisen. Nach dem Herausnehmen des starren Körpers entsteht dann immer noch eine zusammenhängende Leerpackung, so dass die Trägerschale 2 und der Rest der Verpackung während dem Aufreissen der Versiegelung, während dem Herausnehmen und während dem Einbringen des starren Körpers 1 in ein Arbeitsfeld immer mit der gleichen Hand ohne Unterbruch festgehalten werden kann.

### Liste der Bezugszeichen

- 1: starrer Körper
- 2: Trägerschale
- 3: Deckelfolie
- 4: Stützkörper
- 5: Deckel
- 6: Vorsprung
- 7: seitliche Tasche
- 8: Trennwand
- 9: mittige Stützkörper
- 10: Durchbruch
- 11: Schlitz
- 12: Haltelappen
- 13: Gelenk
- 14: Vorsprung
- 15: Kragen

## Patentansprüche

1. Reinstraumverpackung für einen länglichen starren Körper (1), die aus einer Trägerschale (2) aus Kunststoff, welche luftdicht mit einer Deckelfolie (3) verschliessbar ist, und aus Stützkörpern (4, 8, 9) aus kompressiblem Schaumstoff besteht, **dadurch gekennzeichnet,** dass kopf- und fuss-seitige Stützkörper (4, 8) als Trennwand den Kontakt des starren Körpers (1) in Richtung der Längsachse zur Trägerschale (2) verhindern, dass mittige Stützkörper (4, 9) für die Lagerung des starren Körpers (1) in Richtung der Längsachse der Trägerschale (2) jeweils einen Durchbruch (10) aufweisen, der über einen Schlitz (11) auf der Deckelseite zugänglich ist, und dass die Stützkörper (4, 8, 9) quer zur Längsachse der Trägerschale (2) und in Richtung Deckel (5) mit Vorsprüngen (6) in seitlichen Taschen (7) der Trägerschale (2) verankert sind.

2. Verpackung nach Anspruch 1, dadurch gekennzeichnet, dass der Durchbruch (10) der mittigen Stützkörper (4, 9) durch Abklappen von Haltelappen (12) an den mittigen Stützkörpern (4, 9) zum Deckel (5) hin für das Einlegen und Herausnehmen von starren Körpern (1) zugänglich ist.

3. Verpackung nach Anspruch 2, dadurch gekennzeichnet, dass der Widerstand der Haltelappen (12) beim Herausnehmen des starren Körpers (1) in Richtung des Deckels (5) kleiner ist als der Widerstand der Vorsprünge (6) gegen das Herauslösen der Stützkörper (4, 9).

4. Verpackung nach einem der Ansprüche 1 bis 3, dadurch gekennzeichnet, dass bei verschlossener Verpackung die Haltelappen (12) mit ihrer Aussenseite an der Deckelfolie (3) anliegen und mit ihrer Innenseite gegen den starren Körper (1) pressen.

5. Verpackung nach Anspruch 4, dadurch gekennzeichnet, dass bei geschlossener Verpackung die Verspannung der Durchbrüche (10) eine Fixierung des starren Körpers (1) bewirkt.

6. Verpackung nach Anspruch 4 oder 5, dadurch gekennzeichnet, dass die Durchbrüche (10) eine mit Vorsprüngen (14) versehene Oberfläche aufweisen, damit mit der Deformation dieser Vorsprünge (14) Anpresskräfte an dem starren Körper (1) erzeugt werden.

7. Verpackung nach einem der Ansprüche 1 bis 6, dadurch gekennzeichnet, dass der Schaumstoff der Stützkörper (4, 8, 9) an seiner Oberfläche keinerlei lose oder schwach befestigte Partikel aufweist.

8. Verpackung nach einem der Ansprüche 1 bis 7, dadurch gekennzeichnet, dass Trägerschale (2) und Deckelfolie (3) aus transparentem Kunststoff bestehen, um die Kennzeichnung am eingeschlossenen starren Körper (1) im verpackten Zustand ablesen zu können.

## Claims

1. A sterile environment packaging for an oblong rigid solid (1), which consists of a supporting shell (2) made from plastic, which can be sealed with a foil lid (3) so that it is airtight, and of support members (4, 8, 9) made from compressible foam,
**characterised in that** support members (4, 8) at the top and at the base act as partitions to prevent contact between the rigid solid (1) in the direction of the longitudinal axis and the supporting shell (2),
**in that** each central support member (4, 9) for retaining the rigid solid (1) in the direction of the longitudinal axis of the supporting shell (2) has an opening (10), which is accessible via a slit (11) on the lid side,
**and in that** the support members (4, 8, 9) are attached at right angles to the longitudinal axis of the supporting shell (2) and in the direction of the lid (5) with protrusions (6) into lateral pockets (7) in the supporting shell (2).

2. A packaging according to Claim 1,
**characterised in that** the opening (10) in the central support members (4, 9) is accessible by folding back retaining flaps (12) on the central support members (4, 9) towards the lid (5) for the insertion and removal of rigid solids (1).

3. A packaging according to Claim 2,
**characterised in that** the resistance of the retaining flaps (12) when withdrawing the rigid solid (1) in the direction of the lid (5) is less than the resistance of the protrusions (6) against the detachment of the support members (4, 9).

4. A packaging according to one of Claims 1 to 3,
**characterised in that** when the packaging is sealed the retaining flaps (12) abut with their outside against the film lid (3) and press with their inside against the rigid solid (1).

5. A packaging according to Claim 4,
**characterised in that** when the packaging is sealed the bracing of the openings (10) brings about a clamping of the rigid solid (1).

6. A packaging according to Claim 4 or 5,
**characterised in that** the openings (10) comprise a surface provided with protrusions (14) so that contact pressure is produced at the rigid solid (1) with the deformation of these protrusions (14).

7. A packaging according to one of Claims 1 to 6,
**characterised in that** at its surface the foam of the support members (4, 8, 9) does not have any loose or badly attached particles at all.

8. A packaging according to one of Claims 1 to 7,
**characterised in that** supporting shell (2) and film lid (3) are made from transparent plastic so that in the packed condition it is possible to read the marking on the enclosed rigid solid (1).

## Revendications

1. Emballage stérile pour un corps (1) rigide, allongé, qui est constitué d'une coque de support (2) en matière synthétique, laquelle peut être fermée de manière étanche à l'air avec une feuille-couvercle (3), et de corps d'appui (4, 8, 9) en mousse compressible, caractérisé en ce que les corps d'appui (4, 8) côté tête et côté pied en tant que cloisons empêchent le contact du corps rigide (1) en direction de l'axe longitudinal avec la coque de support (2) et en ce que des corps d'appui (4, 9) centraux présentent chacun un ajour (10) pour supporter le corps rigide (1) en direction de l'axe longitudinal de la coque de support (2), lequel ajour est accessible par une fente (11) sur le côté du couvercle et en ce que les corps d'appui (4, 8, 9) sont ancrés, transversalement à l'axe longitudinal de la coque de support (2) et en direction du couvercle (5), avec des parties saillantes (6), dans des poches (7) latérales de la coque de support (2).

2. Emballage selon la revendication 1, caractérisé en ce que l'ajour (10) des corps d'appui (4, 9) centraux est accessible par rabattement de pattes de maintien (12) sur les corps d'appui (4, 9) centraux, vers le couvercle, pour mettre en place et enlever des corps (1) rigides.

3. Emballage selon la revendication 2, caractérisé en ce que la résistance des pattes de maintien (12) lors de l'enlèvement du corps (1) rigide en direction du couvercle (5), est inférieure à la résistance des parties saillantes (6) contre un détachement des corps d'appui (4, 9).

4. Emballage selon l'une des revendications 1 à 3, caractérisé en ce que lorsque l'emballage est fermé, les pattes de maintien (12) s'appliquent avec leur côté extérieur contre la feuille-couvercle (3) et pressent avec leur côté intérieur contre le corps (1) rigide.

5. Emballage selon la revendication 4, caractérisé en ce que, lorsque l'emballage est fermé, la déformation des ajours (10) provoque une fixation du corps (1) rigide.

6. Emballage selon les revendications 4 ou 5, caractérisé en ce que les ajours (10) présentent une surface pourvue de parties saillantes (14), afin que la déformation de ces parties saillantes (14) provoque des forces de pression contre le corps (1) rigide.

7. Emballage selon l'une des revendications 1 à 6, caractérisé en ce que la mousse des corps d'appui (4, 8, 9) ne présente à sa surface aucune particule libre ou faiblement fixée.

8. Emballage selon l'une des revendications 1 à 7, caractérisé en ce que la coque de support (2) et la feuille-couvercle (3) sont en matière synthétique transparente, afin de permettre la lecture de la désignation apposée sur le corps (1) rigide enfermé, à l'état emballé.
